# EUROPÄISCHE PATENTSCHRIFT

(11) **EP 0 506 714 B1**
(45) Veröffentlichungstag und Bekanntmachung des Hinweises auf die Patenterteilung: **02.11.1994**
(21) Anmeldenummer: 91900666.8
(22) Anmeldetag: 21.12.1990
(51) Int. Cl.: C12N 5/00, A61K 39/12

(54) **MATRIX MIT DARAN ADHÄRENT GEBUNDENEN ZELLEN, SOWIE VERFAHREN ZUR PRODUKTION VON VIRUS/VIRUSANTIGEN**
MATRIX WITH ADHERENTLY BOUND CELLS AND PROCESS FOR PRODUCING VIRUSES/VIRUS ANTIGENS
MATRICE AVEC DES CELLULES LIEES PAR ADHERENCE ET PROCEDE DE PRODUCTION DE VIRUS/ANTIGENES VIRAUX

(30) Priorität: 22.12.1989 AT 2928/89
(43) Veröffentlichungstag der Anmeldung: 07.10.1992
(73) Patentinhaber: IMMUNO Aktiengesellschaft, A-1221 Wien (AT)
(72) Erfinder: MUNDT, Wolfgang, A-1080 Wien (AT); BARRETT, Noel, A-3400 Klosterneuburg/Weidling (AT); DORNER, Friedrich, A-1230 Wien (AT); EIBL, Johann, A-1180 Wien (AT)
(74) Vertreter: Wolfram, Gustav, Dipl.-Ing.
(86) Internationale Anmeldenummer: AT9000128
(87) Internationale Veröffentlichungsnummer: WO9109935

(56) Entgegenhaltungen:
- EP-A- 0 066 726
- EP-A- 0 239 648
- FR-A- 2 444 466
- GB-A- 2 059 991
- GB-A- 2 094 832
- GB-A- 2 151 610

## Beschreibung

Die Erfindung betrifft eine Matrix mit daran adhärent gebundenen menschlichen oder tierischen Zellen, sowie ein Verfahren zur Produktion von Virus/Virusantigen, insbesondere von Frühsommer-Meningoenzephalitis-(FSME)-Virus-Antigen.

Infektionen mit dem Virus der Frühsommer-Meningoenzephalitis (FSME) werden seit dem 2. Weltkrieg in Europa beobachtet. In Österreich, in Süddeutschland und in der Tschechoslowakei werden jedes Jahr mehrere hundert Patienten aufgrund einer FSME-Infektion stationär behandelt.

Das FSME-Virus wird der Familie der Flaviviren zugeordnet, der früheren serologischen Gruppe B der Arboviren, die einen Genus der Virusfamilie Togaviridae darstellt.

Gegen eines der wichtigsten und häufigsten Enzephalitis-Erreger beim Menschen, das Japanische Encephalitis-B-Virus, gibt es seit längerer Zeit Totimpfstoffe. Diese Totimpfstoffe werden aus dem Gehirn infizierter Mäuse gewonnen, gereinigt, und sind als sicher und wirksam anerkannt (Hoke et al., N.Engl.J.Med., 319, 608 (1988)).

Seit dem Jahr 1976 ist ein Impfstoff gegen FSME verfügbar und von den Gesundheitsbehörden zugelassen. Zur Herstellung dieses Impfstoffes wird das Virus im Gehirn infizierter Baby-Mäuse angezüchtet, in Hühnerembryonalzellen vermehrt, mit Formalin inaktiviert und dann einem effizienten Reinigungsverfahren unterworfen (Heinz et al., J.Med.Virol., 6, 103 (1980)).

In der Literatur werden eine Reihe von Möglichkeiten zur Vermehrung von Arboviren im Hinblick auf eine mögliche Impfstoffherstellung beschrieben. Die heute am meisten verwendete Methode ist die Beimpfung von Hühnerembryonalfibroblasten mit einem aus dem Maushirn gewonnenen FSME-Saatvirus und die Kultivierung der beimpften Zellen. Diese Methode erfordert eine aufwendige Reinigung des Antigens, um komplexes, heterologes biologisches Material zu entfernen und um bei wiederholter Verabreichung von daraus gewonnenen Impfstoffdosen einen sensitivierenden Effekt bei den Impflingen zu vermeiden.

Für die Bereitstellung der Hühnerembryonalzellen muß von SPF(= specific pathogen free)-Eiern ausgegangen werden. Diese SPF-Eier müssen zur Aufrechterhaltung ihres SPF-Status vor jeder Verwendung einer Vielzahl von langwierigen Untersuchungen unterzogen werden.

Weiters zeigen Hühnerembryonalzellkulturen nur geringe Generationszahlen bei der Weiterzucht, wodurch der Chargengröße Grenzen gesetzt sind, ein schwieriges Sterilhalten der Primärkulturanzucht und keine Konstanz der Qualität der primären Zellen in bezug auf Virusvermehrung und Antigenproduktion.

Diese Nachteile bestehen nicht nur bei den Verfahren zur Produktion von FSME-Virusantigen, sondern bei der Antigenherstellung ganz allgemein.

Die Erfindung stellt sich die Aufgabe, die Produktion von Virus/Virusantigen, insbesondere von FSME-Virus/Virusantigen, so zu verbessern, daß die oben genannten Nachteile beseitigt werden und ein Verfahren zur Züchtung von Virus/Virusantigen in Zellkulturen zur Verfügung zu stellen, das insbesondere eine Produktion im großtechnischen Maßstab erlaubt, wobei gleichzeitig die Kultur auf einfache Weise steril gehalten werden kann. Weiters soll die Abgabe unerwünschter zellulärer Proteine in den Kulturüberstand minimiert werden.

Zur Lösung der beschriebenen Aufgabe wird eine Matrix zur Verfügung gestellt, d.h. ein Trägermaterial, mit daran adhärent gebundenen menschlichen oder tierischen Zellen, wobei die Zellen mit Virus infiziert sind. Die Erfindung beruht auf der Erkenntnis, daß oberflächenabhängige Zellen, die zur Virusvermehrung geeignet sind, selbst in virusinfiziertem Zustand an einer Matrix adhärent gebunden bleiben, über relativ lange Zeit kontinuierlich Virusantigen produzieren und in das Kulturmedium abgeben.

Es ist möglich, die mit infizierten Zellen beladene erfindungsgemäße Matrix einige Tage bei einer Temperatur zwischen 0°C und 8°C aufzubewahren, also unter Bedingungen, bei denen der Zellmetabolismus und damit die Virusproduktion unterbunden sind. Eine derartig aufbewahrte Matrix kann in der Folge problemlos durch Einbringen in ein Kulturmedium und Einstellen der jeweiligen Kultivierungsbedingungen zur Virusantigenproduktion verwendet werden. Die erfindungsgemäße Matrix stellt somit eine Ausgangskultur dar, die mit konstanter Qualität und Aktivität auf Vorrat produziert werden kann, deren Zustand hinsichtlich Sterilität leicht überprüfbar ist und die jederzeit zur Virusantigenproduktion herangezogen werden kann.

Die Bindung der antigenproduzierenden Zellen an den Träger gestattet weiters eine überaus einfache Handhabung der virusinfizierten und produktionsbereiten Zellen. So ist es beispielsweise möglich, die Virus/Virusantigenproduktion kontinuierlich in einem Perfusionsreaktor durchzuführen. Die Abtrennung der Zellen vom antigenhältigen Medium wird durch ihre Bindung an die Matrix wesentlich erleichtert, wodurch die erfindungsgemäße Matrix die Produktion von Virus/Virusantigen im großtechnischen Maßstab vereinfacht.

Eine bevorzugte Ausführungsform der erfindungsgemäßen Matrix besteht darin, daß als adhärent gebundene Zellen Vero-Zellen ATTC CCL 81 vorgesehen sind, welche vorzugsweise zur Produktion von Frühsommer-Meningoenzephalitis-(FSME)-Virus-Antigen vorgesehen sind und daher mit FSME-Virus infiziert sind.

Die an der Matrix adhärent gebundenen Zellen können aber auch mit Flavivirus oder mit Arenavirus infiziert sein.

Als Material für die Matrix hat sich Glas, quervernetztes Dextran, Gelatine oder Kunststoff als gut geeignet erwiesen, wobei die Matrix am besten als Microcarrier ausgebildet ist, dessen Teilchendurchmesser vorzugsweise im Bereich zwischen 100µm und 3000µm liegt. Diese Microcarrier können eine glatte Oberfläche oder eine poröse Strukturierung aufweisen.

Eine weitere zweckmäßige Ausführungsform der erfindungsgemäßen Matrix ist dadurch gekennzeichnet, daß an ihrer Oberfläche pro cm² zwischen 1x10⁵ und 4x10⁵ Zellen adhärent gebunden sind.

Die Erfindung betrifft auch ein Verfahren zur Produktion von Frühsommer-Meningoenzephalitis-(FSME)-Virus-Antigen unter Verwendung der erfindungsgemäßen Matrix, das dadurch gekennzeichnet ist, daß oberflächenabhängige permanente Zellen, vorzugsweise die Vero-Zellen ATCC CCL 81, mit dem FSME-Virus beimpft werden und die Zellen in einem serumfreien Medium unter Aufrechterhaltung ihrer Lebensfähigkeit an einer Matrix adhärent gebunden gehalten werden, um eine Antigenbildung und eine Antigenabgabe an das Medium aufrecht zu erhalten, worauf das antigenhältige Medium von den trägergebundenen Zellen getrennt und in bekannter Weise durch Aufkonzentrieren, Inaktivieren und Reinigen zu einem galenisch akzeptablen Präparat aufgearbeitet wird.

Die Verozellinie ATCC CCL 81 wird aus dem Nierengewebe der grünen Meerkatze (Cercopithecus aethiops) gewonnen und kann in serumfreiem Medium metabolisch aktiv gehalten werden. Für eine solche permanente Zellinie wird eine Muttersaatzellbank und eine Arbeitssaatzellbank einmal angelegt und alle Untersuchungen auf kontaminierende Substanzen durchgeführt. Diese permanente Zellinie ist somit genau charakterisierbar nicht nur im Hinblick auf Freiheit von kontaminierenden Mikroorganismen, sondern auch auf Wachstumsverhalten, Anzucht, Vermehrungsverhalten und - wenn einmal optimiert - als konstant zu betrachten.

Beim erfindungsgemäßen Verfahren werden vorzugsweise Verozellen verwendet, die auf Microcarriern gebunden sind. Dadurch kann eine hohe Zelldichte erreicht werden, die bei den bisher benutzten primären Zellkulturen weder in Rouxflaschen noch in Suspension erreichbar war und eine erhebliche Ausbeutesteigerung an Virus und an Virusantigen pro Fermentationsvolumen ermöglicht.

Eine vorteilhafte Ausgestaltung des erfindungsgemäßen Verfahrens besteht darin, daß die Virusvermehrung und Antigenbildung in einem kontinuierlich betriebenen Perfusionsreaktor während einer Dauer von mindestens 5 Tagen, bei einer Temperatur zwischen 34 und 37°C, durchgeführt wird, wobei die Perfusion mit einer Perfusionsrate von 0,3 bis 10 v/v/Tag durchgeführt werden kann. Im Perfusionsreaktor kann weiters eine eine Zelldichte von 2 x 10⁹ bis 2 x 10¹⁰ Zellen pro Liter Fermentationsvolumen, letztere bei einem Fließbett-Fermenter, vorgesehen werden.

Die erfindungsgemäße Virusvermehrung in einer Perfusionskultur ermöglicht eine im Vergleich zu einer chargenweisen Kultivierung wesentliche Verkürzung der durch die Perfusionsrate vorgegebenen Verweilzeit des Virus- und des Virusantigens im Medium. Durch die kürzere Verveilzeit kommt es zu einer weit geringeren thermischen Inaktivierung und damit zu einer höheren Produktivität des erfindungsgemäßen Verfahrens. So kann im Perfusionsmedium eine Antigenkonzentration von 1 bis 10 µg/ml erreicht und aufrecht erhalten werden.

Beim erfindungsgemäßen Verfahren können auf einfache Weise die zur Kultivierung optimalen Bedingungen eingestellt werden. Außerdem sind zur Durchführung wesentlich geringere Manipulationen erforderlich als bei allen bekannten Verfahren, was eine größere Sicherheit im Umgang mit dem infektiösem Material bedeutet und ein kontinuierliches rasches Aufarbeiten des Virus und der Virusantigene aus dem Kulturmedium ermöglicht.

Die Herstellung des Virusinokulums, die Anzucht der Zellen für die Virus- bzw. Virusantigenproduktion und die eigentliche Virus- bzw. Virusantigenproduktion werden nachstehend näher beschrieben.

### 1. Virusinokulum

Zellen (z.B. Vero ATCC CCL 81) werden in Rollerflaschen bei 37°C bis zur Konfluenz angezüchtet und mit 1 ml einer Saatvirussupension infiziert. Ab dem 2. Tag nach Infektion wird täglich ein halber Medienwechsel mit serumfreiem Medium durchgeführt. Die Medienüberstände vom 4. bis zum 8. Tag enthalten 2-5 x 10⁷ p.f.u. pro ml und werden bis zu ihrer Verwendung als Virusinokulum bei -20°C gelagert.

### 2. Anzucht der Zellen für Virus/Virusantigenproduktion

Ausgehend von den in Flüssigstickstoff gelagerten ATTC CCL 81 Arbeitssaatzellen, wird eine Vermehrung dieser Zellen in Gewebekulturflaschen durchgeführt bis eine Zellmenge erreicht ist, die es erlaubt, einen Fermenter zu inokulieren. Die weitere Kultivierung der Zellen erfolgt in Fermentationsgefäßen bei 37°C, wobei man den adhärent wachsenden Arbeitssaatzellen möglichst viel Oberfläche zum Anhaften zur Verfügung stellen soll. Solche großen Oberflächen bieten sich in der Verwendung von Rollerflaschen aus Glas oder Polystyrol oder in der Verwendung von Microcarriern (MC) an. Am besten sind MC aus quervernetztem Dextran mit einer Größe zwischen 170µm und 250µm geeignet.

Die mit Saatzellen beladenen MC werden bei 37°C kultiviert bis eine Zelldichte von 1.10⁵ - 4.10⁵ Zellen pro cm² erreicht ist. Diese Zelldichte ist im allgemeinen nach sechs Tagen erreicht. Während der Kultivierung kommt es zu einer vollständigen Überwachsung der Microcarrier mit Zellen, wobei schließlich einzelne Microcarrier über den auf ihrer Oberfläche haftenden Zellrasen miteinander zur Gruppen verwachsen können.

### 3. Virus/Virusantigenproduktion

Nachdem die angegebene Zelldichte erreicht wurde, werden zur Herstellung der erfindungsgemäßen Matrix die am MC gebundenen Zellen mit dem Virusinokulum infiziert (1-0,01 pfu/Zelle, bevorzugt 0,1 pfu/Zelle). Die erfindungsgemäße Matrix kann einige Tage bei einer Temperatur zwischen 0°C und 8°C gelagert werden oder sofort zur Virusantigenproduktion verwendet werden.

Zur Antigenproduktion werden die mit infizierten Zellen beladenen MC in einen Perfusionsreaktor eingebracht. Ab diesem Zeitpunkt der Virusinfektion wird in der Kultur nur serumfreies Medium verwendet, das kontinuierlich durch den Perfusionsreaktor gepumpt wird, während die auf den Microcarriern kultivierten Zellen mittels einer Rückhaltevorrichtung im Reaktor zurückgehalten werden. Im ablaufenden Kulturmedium befindet sich ab dem 2. Tag nach Infektion Virusantigen in hoher Konzentration und kann mindestens 10 Tage daraus kontinuierlich gewonnen werden.

Mit den nachfolgenden Beispielen wird das erfindungsgemäße Verfahren noch weiter erläutert. Die Bestimmung des Virusantigens erfolgte in allen Beispielen mit einem Antigen-ELISA.

### Beispiel 1

Vero-Zellen ATCC CCL 81 wurden in einem 6-l-Fermenter auf Microcarrier (Cytodex 3 der Firma Pharmacia) bei 37° C bis zu einer Zellzahl von 2x10⁶ pro ml Kulturmedium (DMEM = Dulbecco's Eagle Medium) kultiviert und
a) mit FSME-Virus (0,1 pfu/Zelle) infiziert und die Virusvermehrung chargenweise durchgeführt

**Tabelle 1**

| Tage nach Infektion | Virus/Virusantigen µg/ml |
|---|---|
| 2 | 0,20 |
| 3 | 0,70 |
| 4 | 1,60 |
| 5 | 2,70 |
| 6 | 4,00 |
| 7 | 3,80 |
| 8 | 2,90 |

Die Produktivität betrug pro l Fermentationsvolumen 4mg Virus/Virusantigen.
b) mit FSME-Virus (0,1 pfu/Zelle) infiziert und Kulturmedium (DMEM) kontinuierlich mit 0,5 Volumen/Fermentervolumen/Tag perfundiert

**Tabelle 2**

| Tage nach Infektion | Virus/Virusantigen µg/ml |
|---|---|
| 2 | 0,30 |
| 3 | 1,60 |
| 4 | 4,50 |
| 5 | 4,50 |
| 6 | 2,50 |
| 7 | 3,20 |
| 8 | 2,90 |
| 9 | 2,50 |
| 10 | 2,30 |

Die Produktivität betrug pro l Fermentationsvolumen 13,7 mg Virus/Virusantigen.
c) mit FSME-Virus (0,1 pfu/Zelle) infiziert und Kulturmedium (DMEM) kontinuierlich mit 1 Volumen/Fermentervolumen/Tag perfundiert

**Tabelle 3**

| Tage nach Infektion | Virus/Virusantigen µg/ml |
|---|---|
| 2 | 0,45 |
| 3 | 1,40 |
| 4 | 2,00 |
| 5 | 2,00 |
| 6 | 1,70 |
| 7 | 1,60 |
| 8 | 1,10 |
| 9 | 1,10 |
| 10 | 0,90 |

Die Produktivität betrug pro l Fermentationsvolumen 12,4 mg Virus/Virusantigen.

### Beispiel 2

Vero-Zellen (ATCC CCL 81) wurden in einem 40-l-Fermenter auf Microcarrier (Cytodex 3 der Firma Pharmacia) bei 37° C bis zu einer Zellzahl von 2x10⁶ Zellen/ml kultiviert und nach Infektion mit FSME-Virus (0,1 pfu/Zelle) kontinuierlich mit Medium (DMEM) (0,33 Vol/Fermentervolumen/Tag) perfundiert.

**Tabelle 4**

| Tage nach Infektion | Virus/Virusantigen µg/ml |
|---|---|
| 2 | 1,60 |
| 3 | 3,50 |
| 4 | 5,00 |
| 5 | 4,30 |
| 6 | 4,00 |
| 7 | 2,90 |
| 8 | 2,70 |
| 9 | 2,10 |
| 10 | 2,00 |

Die Produktivität betrug pro l Fermentationsvolumen 10,7 mg Virus/Virusantigen.

### Beispiel 3

Vero-Zellen (ATCC CCL 81) wurden in einem 40-l-Fermenter auf Microcarrier (Cytodex 3 der Firma Pharmacia) bei 37°C bis zu einer Zellzahl von 3x10⁶ Zellen/ml kultiviert und nach Infektion mit FSME-Virus (0,1 pfu/Zelle) kontinuierlich mit Medium (DMEM) (1 Vol/Fermentervolumen/Tag) perfundiert.

**Tabelle 5**

| Tage nach Infektion | Virus/Virusantigen µg/ml |
|---|---|
| 2 | 1,10 |
| 3 | 3,80 |
| 4 | 3,90 |
| 5 | 3,00 |
| 6 | 2,30 |
| 7 | 2,20 |
| 8 | 2,00 |
| 9 | 3,15 **) |
| 10 | 2,30 |

| | |
|---|---|
| **) Perfusionsrate wurde auf 0,5 V/Fermentervolumen/Tag reduziert | |

Die Produktivität betrug pro l Fermentationsvolumen 21,7 mg Virus/Virusantigen.

### Beispiel 4

Vero-Zellen (ATCC CCL 81) wurden in 150-l-Fermenter auf Microcarrier (Cytodex 3 der Firma Pharmacia) bei 37° C bis 2x10⁶/ml kultiviert und nach Infektion mit FSME-Virus (0,1 pfu/Zelle) kontinuierlich mit Medium (DMEM) (0,33 Vol/Fermentervolumen/Tag) perfundiert.

**Tabelle 6**

| Tage nach Infektion | Virus/Virusantigen µg/ml |
|---|---|
| 2 | 0,20 |
| 3 | 1,90 |
| 4 | 2,40 |
| 5 | 4,80 |
| 6 | 5,40 |
| 7 | 4,10 |
| 8 | 4,40 |
| 9 | 3,20 |
| 10 | 4,50 |

Die Produktivität betrug pro l Fermentationsvolumen 14,7 mg Virus/Virusantigen.

## Patentansprüche (Patentansprüche für folgende(n) Vertragsstaat(en): AT, BE, CH, DE, DK, FR, GB, IT, LI, NL, SE)

1. Serumfreie Zellkultur enthaltend eine Matrix mit daran adhärent gebundenen menschlichen oder tierischen Zellen zur Produktion von Flavi-Virus/Virusantigen oder Arena-Virus/Virusantigen, welche Zellen mit FSME-Virus oder Arena-Virus infiziert sind.

2. Zellkultur nach Anspruch 1, dadurch gekennzeichnet, daß als adhärent gebundene Zellen Vero-Zellen ATCC CCL 81 vorgesehen sind.

3. Zellkultur nach Anspruch 1 oder 2, dadurch gekennzeichnet, daß die Matrix aus Glas, quervernetztem Dextran, Gelatine oder Kunststoff besteht.

4. Zellkultur nach Anspruch 3, dadurch gekennzeichnet, daß die Matrix als Microcarrier ausgebildet ist.

5. Zellkultur nach einem oder mehreren der Ansprüche 1 bis 4, dadurch gekennzeichnet, daß an der Oberfläche der Matrix pro cm² zwischen 1x10⁵ und 4x10⁵ Zellen adhärent gebunden sind.

6. Verfahren zur Produktion von Frühsommer-Meningoenzephalitis-(FSME)-Virus-Antigen unter Verwendung der in den Ansprüchen 1 bis 5 genannten Matrix, dadurch gekennzeichnet, daß oberflächenabhängige permanente Zellen, vorzugsweise die Vero-Zellen ATCC CCL 81, mit dem FSME-Virus beimpft werden und die Zellen in einem serumfreien Medium unter Aufrechterhaltung ihrer Lebensfähigkeit an einer Matrix adhärent gebunden gehalten werden, um eine Antigenbildung in den Zellen und eine Antigenabgabe an das Medium aufrecht zu erhalten, worauf das antigenhältige Medium von den trägergebundenen Zellen getrennt und in bekannter Weise durch Aufkonzentrieren, Inaktiviren und Reinigen zu einem galenisch akzeptablen Präparat aufgearbeitet wird.

7. Verfahren nach Anspruch 6, dadurch gekennzeichnet, daß die Virusvermehrung und Antigenbildung in einem kontinuierlich betriebenen Perfusionsreaktor während einer Dauer von mindestens 5 Tagen bei einer Temperatur zwischen 34 und 37°C durchgeführt wird.

8. Verfahren nach Anspruch 6, dadurch gekennzeichnet, daß die Perfusion mit einer Perfusionsrate von 0,3 bis 10 v/v/Tag durchgeführt wird.

9. Verfahren nach Anspruch 6, dadurch gekennzeichnet, daß im Perfusionsreaktor eine Zelldichte von 2x10⁹ bis 2x10¹⁰ Zellen pro Liter Fermentationsvolumen, letztere bei einem Fließbett-Fermenter, vorgesehen wird.

10. Verfahren nach einem oder mehreren der Ansprüche 6 bis 9, dadurch gekennzeichnet, daß im Medium eine Virus/Virusantigenkonzentration von 1 bis 10 µg/ml aufrecht erhalten wird.

## Patentansprüche (Patentansprüche für folgende(n) Vertragsstaat(en): ES)

1. Verfahren zur Herstellung einer serumfreien Zellkultur enthaltend eine Matrix mit daran adhärent gebundenen menschlichen oder tierischen Zellen zur Produktion von Flavi-Virus/Virusantigen oder Arena-Virus/Virusantigen, dadurch gekennzeichnet, daß
- menschliche oder tierische Zellen adhärent an eine Matrix gebunden werden,
- die menschlichen oder tierischen Zellen vor oder nach der Bindung an die Matrix mit Flavi-Viren oder Arena-Viren infiziert werden und
- die erhaltene Zellkultur in serumfreiem Medium angezüchtet wird.

2. Verfahren nach Anspruch 1, dadurch gekennzeichnet, daß als adhärent gebundene Zellen Vero-Zellen ATCC CCL 81 vorgesehen sind.

3. Verfahren nach Anspruch 1 oder 2, dadurch gekennzeichnet, daß die Matrix aus Glas, quervernetztem Dextran, Gelatine oder Kunststoff besteht.

4. Verfahren nach Anspruch 3, dadurch gekennzeichnet, daß die Matrix als Microcarrier ausgebildet ist.

5. Verfahren nach einem der Ansprüche 1 bis 4, dadurch gekennzeichnet, daß an der Oberfläche der Matrix pro cm² zwischen 1x10⁵ und 4x10⁵ Zellen adhärent gebunden sind.

6. Verfahren zur Produktion von Frühsommer-Meningoenzephalitis-(FSME)-Virus-Antigen unter Verwendung der in den Ansprüchen 1 bis 5 genannten Matrix, dadurch gekennzeichnet, daß oberflächenabhängige permanente Zellen, vorzugsweise die Vero-Zellen ATCC CCL 81, mit dem FSME-Virus beimpft werden und die Zellen in einem serumfreien Medium unter Aufrechterhaltung ihrer Lebensfähigkeit an einer Matrix adhärent gebunden gehalten werden, um eine Antigenbildung in den Zellen und eine Antigenabgabe an das Medium aufrecht zu erhalten, worauf das antigenhältige Medium von den trägergebundenen Zellen getrennt und in bekannter Weise durch Aufkonzentrieren, Inaktivieren und Reinigen zu einem galenisch akzeptablen Präparat aufgearbeitet wird.

7. Verfahren nach Anspruch 6, dadurch gekennzeichnet, daß die Virusvermehrung und Antigenbildung in einem kontinuierlich betriebenen Perfusionsreaktor während einer Dauer von mindestens 5 Tagen bei einer Temperatur zwischen 34 und 37°C durchgeführt wird.

8. Verfahren nach Anspruch 6, dadurch gekennzeichnet, daß die Perfusion mit einer Perfusionsrate von 0,3 bis 10 v/v/Tag durchgeführt wird.

9. Verfahren nach Anspruch 6, dadurch gekennzeichnet, daß im Perfusionsreaktor eine Zelldichte von 2x10⁹ bis 2x10¹⁰ Zellen pro Liter Fermentationsvolumen, letztere bei einem Fließbett-Fermenter, vorgesehen wird.

10. Verfahren nach einem oder mehreren der Ansprüche 6 bis 9, dadurch gekennzeichnet, daß im Medium eine Virus/Virusantigenkonzentration von 1 bis 10 µg/ml aufrecht erhalten wird.

## Claims (Claims for the following Contracting State(s): AT, BE, CH, DE, DK, FR, GB, IT, LI, NL, SE)

1. A serum-free cell culture containing a matrix with human or animal cells adherently bound thereto, for the production of flavivirus/virus antigen or arena virus/virus antigen, which cells are infected with TBE virus or arena virus.

2. A cell culture according to claim 1, characterized in that Vero cells ATCC CCL 81 are provided as said adherently bound cells.

3. A cell culture according to claim 1 or 2, characterized in that the matrix is comprised of glass, cross-linked dextran, gelatine or synthetic material.

4. A cell culture according to claim 3, characterized in that the matrix is a microcarrier.

5. A cell culture according to one or several of claims 1 to 4, characterized in that between 1x10⁵ and 4x10⁵ cells are adherently bound to the surface of the matrix per cm² thereof.

6. A method of producing tick-borne encephalitis (TBE) virus antigen by using the matrix mentioned in claims 1 to 5, characterized in that surface-dependent permanent cells, preferably Vero cells ATCC CCL 81, are inoculated with TBE virus and the cells are kept adherently bound to a matrix in a serum-free medium while maintaining their viability in order to ensure the formation of antigen in the cells and the release of antigen into the medium, whereupon the antigen-containing medium is separated from the carrier-bound cells and is processed to a galenically acceptable preparation in a known manner by concentration, inactivation and purification.

7. A method according to claim 6, characterized in that virus propagation and antigen formation are carried out in a continuously operated perfusion reactor for a period of at least 5 days at a temperature of between 34 and 37°C.

8. A method according to claim 6, characterized in that perfusion is carried out at a perfusion rate of from 0.3 to 10 v/v per day.

9. A method according to claim 6, characterized in that a cell density of from 2x10⁹ to 2x10¹⁰ cells per liter of fermentation volume is provided in the perfusion reactor, the latter density in a fluidized bed fermenter.

10. A method according to one or several of claims 6 to 9, characterized in that a virus/virus antigen concentration of from 1 to 10 µg/ml is maintained in the medium.

## Claims (Claims for the following Contracting State(s): ES)

1. A method of producing a serum-free cell culture containing a matrix with human or animal cells adherently bound thereto, for the production of flavivirus/virus antigen or arena virus/virus antigen, characterized in that
- human or animal cells are adherently bound to a matrix,
- the human or animal cells are infected with flaviviruses or arena viruses prior or after binding to the matrix, and
- the cell culture obtained is cultured in serum-free medium.

2. A method according to claim 1, characterized in that Vero cells ATCC CCL 81 are provided as said adherently bound cells.

3. A method according to claim 1 or 2, characterized in that the matrix is comprised of glass, cross-linked dextran, gelatine or synthetic material.

4. A method according to claim 3, characterized in that the matrix is designed as a microcarrier.

5. A method according to one or several of claims 1 to 4, characterized in that between 1x10⁵ and 4x10⁵ cells are adherently bound to the surface of the matrix per cm² thereof.

6. A method of producing tick-borne encephalitis (TBE) virus antigen by using the matrix mentioned in claims 1 to 5, characterized in that surface-dependent permanent cells, preferably Vero cells ATCC CCL 81, are inoculated with TBE virus and the cells are kept adherently bound to a matrix in a serum-free medium while maintaining their viability in order to ensure the formation of antigen in the cells and the release of antigen into the medium, whereupon the antigen-containing medium is separated from the carrier-bound cells and is processed to a galenically acceptable preparation in a known manner by concentration, inactivation and purification.

7. A method according to claim 6, characterized in that virus propagation and antigen formation are carried out in a continuously operated perfusion reactor for a period of at least 5 days at a temperature of between 34 and 37°C.

8. A method according to claim 6, characterized in that perfusion is carried out at a perfusion rate of from 0.3 to 10 v/v per day.

9. A method according to claim 6, characterized in that a cell density of from 2x10⁹ to 2x10¹⁰ cells per liter of fermentation volume is provided in the perfusion reactor, the latter density in a fluidized bed fermenter.

10. A method according to one or several of claims 6 to 9, characterized in that a virus/virus antigen concentration of from 1 to 10 µg/ml is maintained in the medium.

## Revendications (Revendications pour l'(les) Etat(s) contractant(s) suivant(s): AT, BE, CH, DE, DK, FR, GB, IT, LI, NL, SE)

1. Culture des cellules exempte de sérum et contenant une matrice avec des cellules humaines ou animales y liées adhéremment, pour la production de virus Flavi/antigène de virus ou de virus Arena/antigène de virus, lesquelles cellules sont infectées du virus FSME ou du virus Arena.

2. Culture des cellules selon la revendication 1, caractérisée en ce que des cellules Vero ATCC CCL 81 sont prévues en tant que cellules liées adhéremment.

3. Culture des cellules selon la revendication 1 ou 2, caractérisée en ce que la matrice est constituée de la verre, du dextrane réticulé, de la gélatine ou de la matière synthétique.

4. Culture des cellules selon la revendication 3, caractérisée en ce que la matrice est configurée en tant que microsupport.

5. Culture des cellules selon l'une ou plusieurs des revendications 1 à 4, caractérisée en ce qu'entre 1x10⁵ et 4x10⁵ cellules sont liées adhéremment à la surface de la matrice par cm².

6. Procédé pour la production de l'antigène contre le virus de la méningoencéphalite du début de l'été (FSME) en utilisant la matrice définie dans les revendications 1 à 5, caractérisé en ce que des cellules permanentes dépendentes de surface, de préference les cellules Vero ATCC CCL 81, sont inoculées du virus FSME et les cellules sont tenues réliées adhéramment à une matrice dans un milieu exempte de sérum en maintenant leur viabilité en vue de maintenir la formation d'antigène dans les cellules ainsi que le dégagement d'antigène dans le milieu, aprés quoi le milieu contentant de l'antigène est séparé des cellules liées au support et, à la manière connue, est retraitée à une préparation galéniquement acceptable par concentration, inactivation et purification.

7. Procédé selon la revendication 6, caractérisé en ce que la prolifération de virus et la formation d'antigènes sont effectuées dans un réacteur de perfusion opéré en continu pendant une durée d'au moins 5 jours à une température entre 34 et 37°C.

8. Procédé selon la revendication 6, caractérisé en ce que la perfusion est effectuée à un débit de perfusion de 0,3 à 10 v/v/jour.

9. Procédé selon la revendication 6, caractérisé en ce que, dans le réacteur de perfusion, une densité cellulaire de 2x10⁹ à 2x10¹⁰ cellules par litre de volume de fermentation est prévue, la dernière dans un fermentateur à lit fluidisé.

10. Procédé selon l'une ou plusieurs des revendications 6 à 9, caractérisé une concentration virus/antigène de virus allant de 1 à 10 µg/ml est maintenue dans le milieu.

## Revendications (Revendications pour l'(les) Etat(s) contractant(s) suivant(s): ES)

1. Procédé pour la production d'une culture des cellules exempte de sérum et contenant une matrice avec des cellules humaines ou animales y liées adhéremment, pour la production de virus Flavi/antigène de virus ou de virus Arena/antigène de virus, caractérisé en ce que
- des cellules humaines ou animales sont liées adhéremment à une matrice,
- les cellules humaines ou animales sont infectées des virus Flavi ou des virus Arena avant ou après avoir être liées à la matrice et
- la culture des cellules obtenue est cultivée dans du milieu exempte de sérum.

2. Procédé selon la revendication 1, caractérisé en ce que des cellules Vero ATCC CCL 81 sont prévues en tant que cellules liées adhéremment.

3. Procédé selon la revendication 1 ou 2, caractérisé en ce que la matrice est constituée de la verre, du dextrane réticulé, de la gélatine ou de la matière synthétique.

4. Procédé selon la revendication 3, caractérisée en ce que la matrice est configurée en tant que microsupport.

5. Procédé selon l'une ou plusieurs des revendications 1 à 4, caractérisée en ce qu'entre 1x10⁵ et 4x10⁵ cellules sont liées adhéremment à la surface de la matrice par cm².

6. Procédé pour la production de l'antigène contre le virus de la méningoencéphalite du début de l'été (FSME) en utilisant la matrice mentionnée dans les revendications 1 à 5, caractérisé en ce que des cellules permanentes dépendentes de surface, de préference les cellules Vero ATCC CCL 81, sont inoculées du virus FSME et les cellules sont tenues réliées adhéramment à une matrice dans un milieu exempte de sérum en maintenant leur viabilité en vue de maintenir la formation d'antigène dans les cellules ainsi que le dégagement d'antigène dans le milieu, aprés quoi le milieu contentant de l'antigène est séparé des cellules liées au support et, à la manière connue, est traitée en une préparation galéniquement acceptable par concentration, inactivation et purification.

7. Procédé selon la revendication 6, caractérisé en ce que la prolifération de virus et la formation d'antigènes sont effectuées dans un réacteur de perfusion opéré en continu pendant une durée d'au moins 5 jours à une température entre 34 et 37°C.

8. Procédé selon la revendication 6, caractérisé en ce que la perfusion est effectuée à un débit de perfusion de 0,3 à 10 v/v/jour.

9. Procédé selon la revendication 6, caractérisé en ce que, dans le réacteur de perfusion, une densité cellulaire de 2x10⁹ à 2x10¹⁰ cellules par litre de volume de fermentation est prévue, la dernière densité dans un fermentateur à lit fluidisé.

10. Procédé selon l'une ou plusieurs des revendications 6 à 9, caractérisé en ce qu'une concentration virus/antigène de virus allant de 1 à 10 µg/ml est maintenue dans le milieu.
